# EUROPÄISCHE PATENTSCHRIFT

(11) **EP 2 108 402 B1**
(45) Veröffentlichungstag und Bekanntmachung des Hinweises auf die Patenterteilung: **02.12.2015**
(21) Anmeldenummer: 09154923.8
(22) Anmeldetag: 11.03.2009
(51) Int. Cl.: A61N 5/10

(54) **Verfahren und Vorrichtung zum Erstellen eines Bestrahlungsplans**
Method and device producing a radiation plan
Procédé et dispositif destinés à l'établissement d'un plan de rayonnement

(30) Priorität: 10.04.2008 DE 102008018417
(43) Veröffentlichungstag der Anmeldung: 14.10.2009
(73) Patentinhaber: Siemens Aktiengesellschaft, 80333 München (DE)
(72) Erfinder: Lomax, Tony, 5210, Windisch (CH); Negreanu-Macian, Cezarina, 8400, Winterthur (DE)

(56) Entgegenhaltungen:
- PFLUGFELDER D ET AL: "Quantifying lateral tissue heterogeneities in hadron therapy" MEDICAL PHYSICS, AIP, MELVILLE, NY, US, Bd. 34, Nr. 4, 28. März 2007 (2007-03-28), Seiten 1506-1513, XP012103387 ISSN: 0094-2405
- JIN L ET AL: "Investigation of optimal beam margins for stereotactic radiotherapy of lung-cancer using Monte Carlo dose calculations; Optimal beam margins for lung-cancer SBRT" PHYSICS IN MEDICINE AND BIOLOGY, TAYLOR AND FRANCIS LTD. LONDON, GB, Bd. 52, Nr. 12, 21. Juni 2007 (2007-06-21), Seiten 3549-3561, XP020112925 ISSN: 0031-9155
- RIETZEL E ET AL: "Four-dimensional image-based treatment planning: Target volume segmentation and dose calculation in the presence of respiratory motion" INTERNATIONAL JOURNAL OF RADIATION: ONCOLOGY BIOLOGY PHYSICS, PERGAMON PRESS, US, Bd. 61, Nr. 5, 1. April 2005 (2005-04-01), Seiten 1535-1550, XP025262900 ISSN: 0360-3016 [gefunden am 2005-04-01]
- JAEKEL O ET AL: "TREATMENT PLANNING FOR HEAVY ION IRRADIATION" PHYSICA MEDICA, ACTA MEDICA EDIZIONI E CONGRESSI, ROME, IT, Bd. 14, Nr. 1, 1. Januar 1997 (1997-01-01), Seiten 53-62, XP000908768 ISSN: 1120-1797

## Beschreibung

Die Erfindung betrifft ein Verfahren und eine Vorrichtung zum Erstellen eines Bestrahlungsplans, wie sie insbesondere im Rahmen der Planung einer Bestrahlung eines erkrankten Gewebes mit einem Behandlungsstrahl eingesetzt werden.

Die Strahlentherapie gehört zu den etablierten Methoden insbesondere zur Behandlung von Tumorerkrankungen. Hierbei werden Strahlen auf ein zu bestrahlendes Zielvolumen eines Körpers gerichtet. Im Rahmen der Partikeltherapie, einer Sonderform der Strahlentherapie, werden beispielsweise Protonen, Kohlenstoffionen oder andere Ionen auf hohe Energien beschleunigt und auf das zu bestrahlende Gewebe gerichtet. Es können aber auch andere Strahlenarten wie beispielsweise Elektronen- oder Röntgenstrahlen zur Bestrahlung verwendet werden. Bestrahlungsverfahren, wie sie in der Strahlentherapie oder Partikeltherapie eingesetzt werden, können jedoch auch in nicht-therapeutischen Gebieten Anwendung finden kann. Hierzu gehören beispielsweise Forschungsarbeiten im Rahmen der Partikeltherapie, die an nicht-lebenden Phantomen oder Körpern durchgeführt werden, Bestrahlungen von Materialien, etc.

Um ein Zielvolumen möglichst genau zu bestrahlen, ist es wichtig, dass der Behandlungsstrahl seine Energie möglichst nur im Zielvolumen abgibt und umliegendes Gewebe geschont wird. Um dies zu erreichen, wird üblicherweise vor der Bestrahlung eines Zielvolumens ein so genannter Bestrahlungsplan erstellt, in welchem die Parameter, mit der die Bestrahlung stattfinden soll, genau festgelegt werden.

Verschiedene Faktoren können dazu führen, dass die Bestrahlung eines Zielvolumens nicht in allen Details genau planbar und vorhersagbar ist und geringfügig von der geplanten Ideal-Bestrahlung abweichen kann. Gründe hierfür sind zum Beispiel eine Bewegung des Zielvolumens, Ungenauigkeiten bei der Positionierung des Zielvolumens in Bezug auf den Behandlungsstrahl, Ungenauigkeiten bei der Durchführung der Planung, Veränderungen des Zielvolumens zwischen dem Planungszeitpunkt und dem Behandlungszeitpunkt, etc.

Es wird daher versucht, diese Ungenauigkeiten und Unwägbarkeiten bei der Planung zu berücksichtigen, um im Endergebnis eine möglichst optimale Bestrahlung zu erhalten. Beispielsweise ist bekannt, um das Zielvolumen einen Sicherheitssaum zu legen, damit das Zielvolumen in jedem Fall ausreichend bestrahlt wird.

Der Konferenzbeitrag "Factors affecting the selection of beam directions in proton therapy", Lomax T, 44th AAPM Annual Meeting, Montreal, July 2002, AbstractID 7798, offenbart, dass ein Index, mit dem Dichte-Heterogenitäten quantifiziert werden, die Größe von Effekten vorhersagen kann, die einen Behandlungsplan verschlechtern können.

Die Schrift Pflugfelder D et al., "Quantifying lateral tissue heterogeneities in hadron therapy", Medical Physics, 2007, 34(4), 1506-1513, offenbart ein Verfahren zur Quantifizierung von lateralen Gewebe-Heterogenitäten bei einem gescannten Partikelstrahl. Dabei wird jeder einzelne applizierte Partikelstrahl mit einem Index bewertet, und die Vielzahl der Indices wird bei der Optimierung der Bestrahlungsplanung verwendet.

Es ist die Aufgabe der Erfindung, ein Verfahren und eine Vorrichtung zur Erstellung eines Bestrahlungsplans anzugeben, mit dem es möglich ist, einen Bestrahlungsplan zu erstellen, der robust gegenüber Ungenauigkeiten ist, der eine weitgehend zu den Bestrahlungsplan konforme tatsächliche Bestrahlung ermöglicht und der gleichzeitig mit günstigem Rechenaufwand ermittelt werden kann.

Die Aufgabe wird gelöst durch die Gegenstände der unabhängigen Ansprüche. Vorteilhafte Ausgestaltungen des Verfahrens bzw. der Vorrichtung finden sich in den abhängigen Ansprüchen.

Das Verfahren zur Erstellung eines Bestrahlungsplans umfasst folgende Schritte:
- Vorgeben eines Datensatzes, in welchem ein zu bestrahlendes Objekt repräsentiert ist,
- Bestimmen eines zu bestrahlenden Zielvolumens in dem Objekt,
- Ermitteln eines Maßes, das eine Dichte-Heterogenität für einen Bereich, der von dem geplanten Behandlungsstrahl getroffen wird, kennzeichnet,
- Bestimmen eines Sicherheitssaumes für das zu bestrahlende Zielvolumen in Abhängigkeit des ermittelten Maßes.

Die entsprechende Vorrichtung zur Erstellung eines Bestrahlungsplans weist eine Rechnereinheit auf, eine Eingabevorrichtung sowie eine Ausgabevorrichtung, und ist dazu ausgebildet, die oben genannten Verfahrensschritte durchzuführen.

Ein Bestrahlungsplan kann nun basierend auf dem Sicherheitssaum, der in Abhängigkeit des ermittelten Maßes bestimmt worden ist, erstellt werden.

Der Datensatz - üblicherweise ein CT-Datensatz (CT für Computertomographie) - bildet das zu bestrahlende Objekt üblicherweise dreidimensional ab. Der Datensatz bildet die Grundlage für die folgende Bestrahlungsplanung. Ein Anwender legt üblicherweise interaktiv das zu bestrahlende Zielvolumen in dem Objekt fest. Dies kann manuell, halbautomatisch oder automatisch erfolgen, z.B. unter Zuhilfenahme bekannter Segmentierungsalgorithmen.

Anschließend wird für einen geplanten Behandlungsstrahl das Maß ermittelt. Dies erfolgt unter der Zuhilfenahme der im Datensatz gespeicherten Daten. Das Maß stellt folglich einen Index dar, der die Dichte-Heterogenität in einem Bereich bewertet, welcher von dem geplanten Behandlungsstrahl getroffen würde.

Dieses Maß wird anschließend bei der Bestimmung des Sicherheitssaumes für das zu bestrahlende Zielvolumen verwendet. Dem Verfahren liegt dabei die Idee zu Grunde, dass, je größer die Heterogenität des durch den Behandlungsstrahls getroffenen Gebiets ist, desto größer ist die Unsicherheit, mit der das zu bestrahlende Gewebe mit einer Dosis durch einen geplanten Behandlungsstrahl belastet wird. Es ist dabei erkannt worden, dass die Dichte-Heterogenität in dem von dem Behandlungsstrahl getroffenen Gebiet Einfluss auf die Homogenität der Zieldosis innerhalb des Zielvolumens, auf die Überdeckung des Zielvolumens mit der deponierten Dosis und/oder auf die Empfindlichkeit einer Dosisverteilung gegenüber einer räumlichen Lageunsicherheit des Zielvolumens hat. Diese Effekte haben Einfluss auf die Wahl des Sicherheitssaumes, der das Zielvolumen umgibt.

Bei dem Verfahren wird nun der Sicherheitssaum, z.B. die Größe, Breite und/oder Ausdehnung des Sicherheitssaumes, in Abhängigkeit des Maßes gewählt. Hierdurch werden die oben genannten Effekte automatisch berücksichtigt. Je größer z.B. die Unsicherheit ist, mit der das Zielvolumen tatsächlich mit einer Dosis belastet wird, bedingt durch eine hohe Dichte-Heterogenität, desto größer kann beispielsweise der Sicherheitssaum von vornherein bei der Bestrahlungsplanung gewählt werden, um zu gewährleisten, dass eine gewünschte minimale Dosisbelastung des Zielvolumens auf jeden Fall erreicht wird.

Dabei kann beispielsweise das Maß für das gesamte Gebiet ermittelt werden, das durch den Behandlungsstrahl getroffen würde. D.h., dass ein einziges Maß die Dichte-Heterogenität in dem gesamten Gebiet bewertet.

Es können aber auch nur Teile von dem gesamten Gebiet berücksichtigt werden. Beispielsweise kann das Maß für den Bereich ermittelt werden, der in Strahlrichtung vor dem Zielvolumen, d.h. im Einfallskanal vor dem Zielvolumen liegt. Wenn beispielsweise Homogenitätsschwankungen der deponierten Dosis im Zentrum des Zielvolumens als weniger relevant angesehen werden, können bei der Ermittlung des Maßes lediglich die Randbereiche des Gebiets, das durch den Behandlungsstrahl getroffen wird, bezüglich ihrer Dichte-Heterogenität bewertet werden.

Insbesondere bei Scanverfahren mit einem Partikelstrahl, bei der die zu deponierende Dosis durch eine Vielzahl von nacheinander zu applizierenden, einzelnen Partikelstrahlen (so genannte "beamlets") erreicht wird, kann der Bereich, dessen Dichte-Heterogenität durch das Maß gekennzeichnet wird, ein Gebiet umfassen, das zumindest von einer Mehrzahl der nacheinander zu applizierenden Partikelstrahlen getroffen wird. Es wird folglich nicht die Heterogenität im Gebiet eines jeden einzelnen Partikelstrahls bewertet, sondern die Heterogenität in einem größeren Gebiet. Insbesondere für die nachfolgenden Verfahrensschritte ist dies vorteilhaft, da eine Vielzahl von Heterogenität-Indices, die die Heterogenität im Zielgebiet eines jeden einzelnen beamlets getrennt bewerten, im weiteren Verlauf der Erstellung und/oder Optimierung des Bestrahlungsplans einen hohen Rechenaufwand bedeuten.

Zusätzlich zu dem Sicherheitssaum kann das Maß dazu verwendet werden, einen Eintrittswinkel des Behandlungsstrahls in das Zielvolumen zu bestimmen bzw. zu optimieren. Beispielsweise kann ein bestimmter Eintrittswinkel des Behandlungsstrahls verworfen werden, wenn das zugehörige Maß, das die Dichte-Heterogenität bewertet, über einem bestimmten Schwellenwert liegt.

Dem Verfahren kann ein Überprüfungsschritt folgen. Dabei kann eine Dosisverteilung, die durch den geplanten Behandlungsstrahl gegeben ist, auf ihre Robustheit gegenüber einer Fehlbestrahlung überprüft werden, d.h. gegenüber einer räumlichen Ungenauigkeit bei der Deposition der gewünschten Bestrahlungsdosis. Gegebenenfalls kann die Bestrahlungsplanung daraufhin geändert werden. Dabei kann z.B. festgestellt werden, dass ein Bestrahlungsplan nicht robust genug gegenüber einer derartigen Ungenauigkeit ist. Das bedeutet, dass die tatsächlich deponierte Dosis beim Auftreten einer derartigen Ungenauigkeit zu stark von der gewünschten Bestrahlungsdosis abweicht. In diesem Falle kann beispielsweise der Bestrahlungsplan geändert werden, z.B. durch eine andere Wahl des Sicherheitssaumes, durch einen anderen Einfallswinkel oder durch andere Veränderungen, solange, bis der Bestrahlungsplan robust genug gegenüber einer räumlichen Ungenauigkeit der Dosis-Deposition ist. In diesem Fall führt eine Ungenauigkeit nur zu einer geringfügig veränderten Dosisverteilung, deren Abweichung von einer gewünschten Bestrahlungsdosis tolerierbar ist.

Die beschriebenen Maßnahmen sind bei verschiedenen Scanverfahren mit einem Partikelstrahl verwendbar:
Bei dem so genannten Spotscanverfahren verweilt der Partikelstrahl an jedem Zielpunkt für eine vorbestimmte Zeit und/oder deponiert an jedem Zielpunkt eine vorbestimmte Anzahl an Partikeln und wird ausgeschaltet, während Ablenkmagnete etc. zum Richten des Partikelstrahls auf einen nächsten Zielpunkt eingestellt werden. Bei dem so genannten Rasterscan-Verfahren verweilt der Partikelstrahl an jedem Zielpunkt während einer vorbestimmten Zeitdauer oder deponiert an jedem Zielpunkt eine vorbestimmte Anzahl an Partikeln, wird aber zwischen den Zielpunkten nicht - oder nicht immer - ausgeschaltet.
Bei so genannten kontinuierlichen Scanverfahren bilden die Zielpunkte zusammenhängende Linien, bilden also kontinuierliche (oder quasikontinuierliche) Mengen, wobei ihre Anzahl abzählbar unendlich ist. Der Partikelstrahl wird bei einem kontinuierlichen Scanverfahren zumindest innerhalb einer Linie bzw. Zeile in eine Isoenergieschicht kontinuierlich abgelenkt und überstreicht die Zielpunkte, ohne an einzelnen Orten zu verweilen. Mit einer Tiefenmodulationsvorrichtung kann auch ein kontinuierliches Scanverfahren durchgeführt werden, bei dem kontinuierlich die Eindringtiefe des Partikelstrahls moduliert wird.
Bei einer Variante des Verfahrens zum Erstellen eines Bestrahlungsplans umfasst das Verfahren folgende Schritte:
   - Vorgeben eines Datensatzes, in welchem ein zu bestrahlendes Objekt repräsentiert ist,
   - Bestimmen eines zu bestrahlenden Zielvolumens in dem Objekt,
   - Vorgeben von zumindest zwei Bestrahlungsfeldern, mit denen das Zielvolumen jeweils aus einer anderen Richtung bestrahlt wird,
   - für jedes Bestrahlungsfeld Ermitteln eines Maßes, das eine Dichte-Heterogenität für einen Bereich, der bei der geplanten Bestrahlung mit dem jeweiligen Bestrahlungsfeld getroffen wird, kennzeichnet,
   - Bestimmen einer Gewichtung der zumindest zwei Bestrahlungsfelder in Abhängigkeit der ermittelten Maße.

Die entsprechende Vorrichtung zur Erstellung eines Bestrahlungsplans weist eine Rechnereinheit auf, eine Eingabe Vorrichtung sowie eine Ausgabevorrichtung, und ist dazu ausgebildet, die eben genannten Verfahrensschritte durchzuführen.

Ein Bestrahlungsplan kann nun basierend auf der Gewichtung, die in Abhängigkeit des ermittelten Maßes bestimmt worden ist, erstellt werden.

Bei diesem Verfahren erfolgt die Erstellung des Bestrahlungsplans mit zumindest zwei Bestrahlungsfeldern, das heißt mit einem Behandlungsstrahl, der jeweils aus einer anderen Richtung auf das Zielvolumen gerichtet wird. Dies kann vorteilhaft sein, z.B. um eine gewünschte Dosis im Zielvolumen zu erreichen bei gleichzeitiger Aussparung von Gebieten, die nicht bestrahlt werden sollen.

Bei den zwei Bestrahlungsfeldern werden somit jeweils unterschiedliche Bereiche im Objekt durch den Behandlungsstrahl getroffen. Für jedes dieser Bestrahlungsfelder wird nun das Maß ermittelt, dass die Dichte-Heterogenität in dem jeweiligen durch den Behandlungsstrahl getroffenen Bereich bewertet. Die ermittelten Maße werden bei diesem Verfahren nun dafür verwendet, eine Gewichtung der Bestrahlungsfelder untereinander zu bestimmen. Wenn beispielsweise ein erstes Bestrahlungsfeld ein Maß aufweist, das durch eine hohe Dichte-Heterogenität gekennzeichnet ist, und ein zweites Bestrahlungsfeld ein Maß aufweist, das durch eine niedrige Dichte-Heterogenität gekennzeichnet ist, kann dies bei der Bestimmung der Gewichtung der beiden Bestrahlungsfelder berücksichtigt werden. Auf diese Weise kann z.B. das Bestrahlungsfeld mit der hohen Dichte-Heterogenität weniger stark gewichtet werden. So können die Unsicherheiten, die bei der Bestrahlung des Bestrahlungsfeldes mit der hohen Dichte-Heterogenität auftreten, verringert werden.

Die Bestimmung der Gewichtung zwischen mehreren Bestrahlungsfeldern nach diesem Verfahren schließt nicht aus, dass zusätzlich zu diesen Bestrahlungsfeldern weitere Bestrahlungsfelder geplant werden, deren Gewichtung nicht anhand des Heterogenitäts-Maßes festgelegt wird. Die Gewichtung dieser weiteren Bestrahlungsfelder kann beispielsweise fest vorgegeben werden oder es können andere Größen als das Heterogenitäts-Maß zur Ermittlung der Gewichtung herangezogen werden.

Wenn bei einem der Bestrahlungsfelder das Maß ermittelt wird, kann beispielsweise das Maß für das gesamte Gebiet ermittelt werden, das durch den Behandlungsstrahl dieses Bestrahlungsfeldes getroffen würde. D.h., dass ein einziges Maß die Heterogenität in dem gesamten Gebiet bewertet.

Es können aber auch nur Teile von dem gesamten Gebiet mit dem Maß bewertet werden. Beispielsweise kann bei einem Bestrahlungsfeld das Maß für den Bereich ermittelt werden, der in Strahlrichtung vor dem Zielvolumen, d.h. im Einfallskanal vor dem Zielvolumen liegt. Wenn beispielsweise Homogenitätsschwankungen der deponierten Dosis im Zentrum des Zielvolumens als weniger relevant angesehen werden, können bei der Ermittlung des Maßes lediglich die Randbereiche des durch den Behandlungsstrahl getroffenen Gebiets hinsichtlich ihrer Dichte-Heterogenität bewertet werden.

Auch hier kann dem Verfahren ein Überprüfungsschritt folgen. Dabei kann ein erstellter Bestrahlungsplan oder ein geplantes Bestrahlungsfeld auf seine Robustheit gegenüber einer Fehlbestrahlung, die auf einer räumlichen Ungenauigkeit der Deposition der gewünschten Bestrahlungsdosis beruht, überprüft und gegebenenfalls geändert werden. Dabei kann z.B. festgestellt werden, dass ein Bestrahlungsplan oder ein Bestrahlungsfeld nicht robust genug gegenüber einer derartigen Ungenauigkeit ist. Das bedeutet, dass die tatsächlich deponierte Dosis beim Auftreten einer derartigen Ungenauigkeit zu stark von der gewünschten Bestrahlungsdosis bzw. deren Verteilung abweicht. In diesem Falle kann beispielsweise der Bestrahlungsplan oder das Bestrahlungsfeld geändert werden, z.B. durch eine andere Wahl des Sicherheitssaumes, durch einen anderen Einfallswinkel oder durch andere Veränderungen, solange, bis der Bestrahlungsplan bzw. das Bestrahlungsfeld robust genug gegenüber einer räumlichen Ungenauigkeit der Dosis-Deposition ist. In diesem Fall führt eine räumliche Ungenauigkeit nur zu einer geringfügig veränderten Dosisverteilung, deren Abweichung von einer gewünschten Bestrahlungsdosis bzw. Verteilung tolerierbar ist.

Insbesondere bei Scanverfahren mit einem Partikelstrahl, bei der bei einem Bestrahlungsfeld die zu deponierende Dosis durch eine Vielzahl von einzelnen Partikelstrahlen (so genannte "beamlets") erreicht wird, die nacheinander aus der gleichen Richtung an verschiedenen Zielpunkten im Zielvolumen appliziert werden, umfasst der Bereich, dessen Dichte-Heterogenität durch das Maß für eines der Bestrahlungsfelder gekennzeichnet wird, ein Gebiet, das zumindest von einer Mehrzahl der nacheinander zu applizierenden Partikelstrahlen getroffen wird - üblicherweise das Gebiet, das durch alle bei einem Bestrahlungsfeld nacheinander zu applizierenden Partikelstrahlen getroffen wird. Auch hier wird folglich nicht die Heterogenität im Gebiet eines jeden einzelnen Partikelstrahls bewertet, sondern die Heterogenität in einem größeren Gebiet. Hieraus ergeben sich die oben erläuterten Vorteile.

Nach dem ein Bestrahlungsplan erstellt worden ist können in einer Weiterbildung der Verfahren anschließend Steuerparameter zum Steuern der Bestrahlungsanlage gemäß dem Bestrahlungsplan ermittelt werden. Anschließend kann eine Steuerung der Bestrahlungsanlage mit den ermittelten Steuerparametern erfolgen, um ein Objekt, für das der Bestrahlungsplan erstellt worden ist, gemäß diesem zu bestrahlen.

In analoger Weise liegt einer Weiterbildung in einer Vorrichtung zur Bestimmen von Steuerparametern einer Bestrahlungsanlage, welche Vorrichtung basierend auf dem erstellten Bestrahlungsplan die Steuerparameter zum Steuern der Bestrahlungsanlage gemäß den Vorgaben des Bestrahlungsplans ermittelt. Eine andere Weiterbildung liegt in der Bestrahlungsanlage, welche ausgebildet ist zum Bestrahlen des Objekts gemäß dem erstellten Bestrahlungsplan, z.B. mithilfe der Steuerparameter, die durch die Vorrichtung zum Bestimmen von Steuerparametern ermittelt worden sind. Die Bestrahlungsanlage kann die Vorrichtung zum Erstellen eines Bestrahlungsplans und/oder die Vorrichtung zum Bestimmen von Steuerparametern einer Bestrahlungsanlage umfassen.

Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Es zeigen:
Fig. 1 einen schematischen Überblick über eine Partikeltherapieanlage,
Fig. 2 ein Diagramm, an der ein zu Grunde liegende Konzept der Erfindung veranschaulicht wird,
Fig. 3 ein zu bestrahlendes Zielvolumen mit einem geplanten Bestrahlungsfeld,
Fig. 4 ein zu bestrahlendes Zielvolumen mit mehreren geplanten Bestrahlungsfeldern,
Fig. 5 eine schematische Übersicht über einzelne Verfahrensschritte.

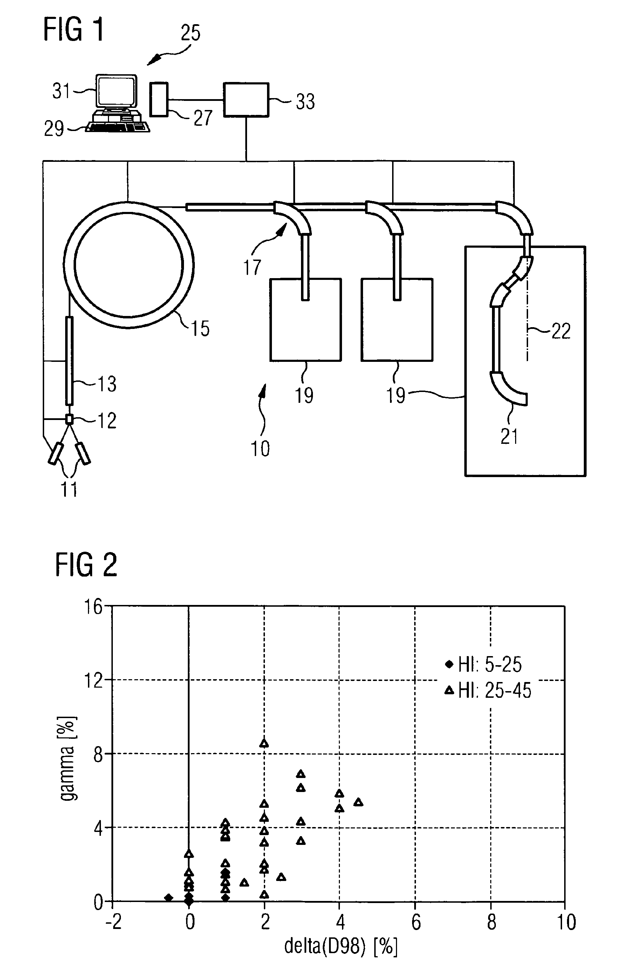

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Behandlungsräume 19. In einem Behandlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Üblicherweise kommt in einer Partikeltherapieanlage 10 eine Vorrichtung 25 zum Erstellen eines Bestrahlungsplans zum Einsatz. Eine derartige Vorrichtung weist üblicherweise eine Rechnereinheit 27 auf, mit einer Eingabevorrichtung 29 und eine Ausgabevorrichtung 31 zur Interaktion mit einem Anwender. Nach Erstellung eines Bestrahlungsplans kann eine Steuervorrichtung 33 zum Steuern der Anlage Steuerparameter gemäß dem erstellten Bestrahlungsplan ermitteln und die Anlage entsprechend steuern. Die Vorrichtung zum Erstellen eines Bestrahlungsplans kann dabei zur Partikeltherapieanlage 10 gehören oder auch als separate Einheit ausgebildet sein.

Der anhand der Figur 1 dargestellte Aufbau einer Partikeltherapieanlage 10 ist im Stand der Technik bekannt und typisch für viele Partikeltherapieanlagen, kann aber auch hiervon abweichen.

Die nachfolgend beschriebenen Ausführungsbeispiele können in derartigen oder ähnlichen Partikeltherapieanlagen Einsatz finden oder aber auch allgemein in medizinischen Anlagen.

Fig. 2 zeigt ein Diagramm, anhand dessen das Konzept erläutert wird, das verschiedenen Ausführungsformen zu Grunde liegt.

Bei der Erstellung von Bestrahlungsplänen wurden verschiedene Bestrahlungsfelder / ganze Bestrahlungspläne getestet. Bei dieser Testung wurde jedes der Bestrahlungsfelder mit einem Heterogenität-Index bewertet, der die Dichte-Heterogenität kennzeichnet, die durch ein Bestrahlungsfeld "gesehen" wird. Die Dichte-Heterogenität wurde dabei für jedes Bestrahlungsfeld durch einen einzigen Heterogenität-Index gekennzeichnet. Je niedriger der Heterogenität-Index war, desto homogener war die Anatomie, durch die das Bestrahlungsfeld trat, desto homogener war folglich die Anatomie im Eintrittskanal eines Bestrahlungsfeldes. Die Robustheit eines Bestrahlungsfeldes/Bestrahlungsplans wurde daraufhin getestet, indem verschiedene Fehler-Dosis-Verteilungs-Datensätze (engl.: "error dose distibution files") ausgewertet wurden. Fehler-DosisVerteilung-Datensätze sind dabei erneut berechnete Dosisverteilungen eines Bestrahlungsfeldes/Bestrahlungsplans unter Berücksichtigung verschiedener Unsicherheiten. Beim Testen der Robustheit wurden alle Fehler-Dosis-Verteilungen unter Verwendung einer normalen Wahrscheinlichkeitsverteilung kombiniert, wobei anschließend die resultierende Verteilung in zweifacher Hinsicht analysiert wurde. Ein erstes Mal erfolgte die Bewertung mit dem so genannten bekannten Gamma-Index, der zur Bewertung der Qualität eines Bestrahlungsplans eingesetzt wird, und ein zweites Mal durch Vergleich der D98-Region (Bereich, indem die deponierte Dosis mindestens 98% der gewünschten Dosis beträgt) mit dem zu bestrahlenden Zielvolumen (deltaD98). Dabei wurden zufällige räumliche Fehler von 2 mm und 4 mm berücksichtigt, zusammen mit dem Effekt, der sich durch eine Veränderung des Sicherheitssaumes von 2 mm bis 5 mm ergibt. Zusätzlich wurden systematische Reichweitenunsicherheiten von +/-3% in die Analyse einbezogen.

Für verschiedene Bestrahlungsfelder mit unterschiedlichen Heterogenität-Indices (HI) wurde in Diagramm der Fig. 2 der ermittelte Gamma-Index (y-Achse) gegenüber dem ermittelten deltaD98-Wert (x-Achse) aufgetragen. Genauer erläutert ist auf der y-Achse die Prozentzahl der Zielpunkte eines Bestrahlungsfeldes aufgetragen, die einen Gamma-Index > 1 aufweisen (Akzeptanz-Kriterium bei der Berechnung der Gamma-Indices für die Zielpunkte: 3%/3 mm).

Bestrahlungsfelder mit einem HI > 25 sind dabei im Diagramm mit offenen, dreieckigen Symbolen dargestellt, Bestrahlungspläne mit einem HI < 25 sind im Diagramm mit geschlossenen, rautenförmigen Symbolen dargestellt. Bestrahlungsfelder mit einem großen HI zeigen deutlich höhere Gamma-Werte und gleichzeitig eine leichte Verschiebung zu höheren deltaD98-Werten hin. Dies bedeutet, dass Dichte-Heterogenitäten einen Einfluss haben auf die Dosisbelastung des gesamten Zielvolumens, jedoch in geringerem Maß auf die Überdeckung des Zielvolumens. Dabei ist erkannt worden, dass eine Veränderung des Sicherheitssaumes im Allgemeinen den deltaD98-Wert reduziert, aber einen geringeren Einfluss auf die Gamma-Analyse hat.

Mit derartigen Analyse-Methoden kann die Rolle von systematischen und zufälligen Fehlern bei der Erstellung eines Bestrahlungsplans ermittelt werden. Zudem ist anhand derartiger Überlegungen erkannt worden, dass beispielsweise ein größerer Sicherheitssaum bei Bestrahlungsfeldern mit einem hohen Heterogenität-Index gewählt werden kann, um eine ausreichende Überdeckung des Zielvolumens (günstiger deltaD98-Wert) zu erreichen. Ebenso kann beispielsweise der Einfallswinkel eines Bestrahlungsfeldes auf das Zielvolumen derart gewählt werden, dass bei dem resultierenden Bestrahlungsfeld ein möglichst kleiner Heterogenität-Index auftritt. Wenn mehrere Bestrahlungsfelder in einen Bestrahlungsplan angewendet werden, kann bei der Bestimmung der Gewichtung der einzelnen Bestrahlungsfelder der Heterogenität-Index ebenso einfließen. Beispielsweise kann das Bestrahlungsfeld mit dem größeren Heterogenität-Index weniger stark gewichtet werden als das Bestrahlungsfeld mit dem kleineren Heterogenität-Index. Auf diese Weise kann das Zielvolumen genauer bestrahlt werden bei besserer Schonung des umliegenden Gewebes. Zudem kann man dadurch einen Bestrahlungsplan erhalten, der insgesamt robuster gegenüber Unsicherheiten ist.

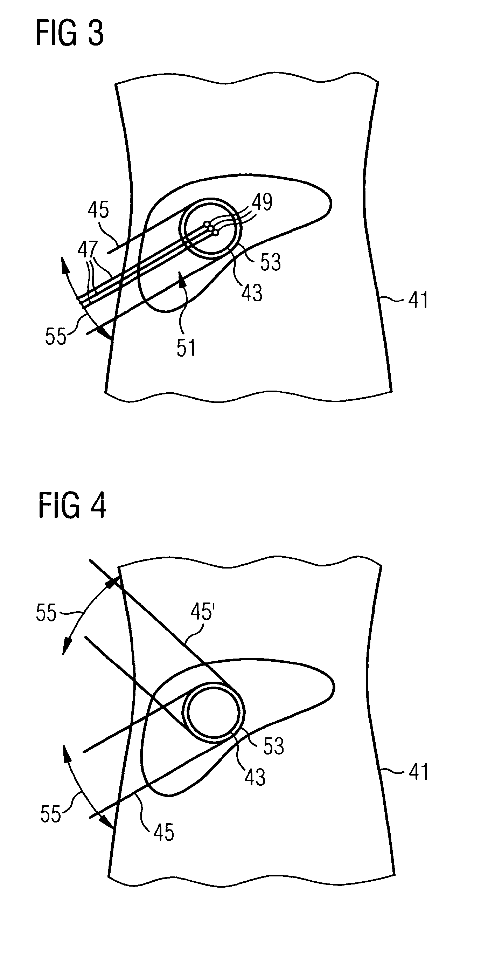

Anhand von Fig. 3 ist die Planung einer Bestrahlung mit einem Bestrahlungsfeld gezeigt.

Die Darstellung des zu bestrahlenden Objekts 41 bzw. des zu bestrahlenden Zielvolumens 43 beruht auf einem Planungsdatensatz, der z.B. durch eine Computertomographie gewonnen wurde. Anhand einer Abbildung, die aus dem Planungsdatensatz gewonnen wurde und die einem Anwender dargestellt wird, kann das zu bestrahlende Zielvolumen 43 in dem zu bestrahlenden Objekt 41 festgelegt werden.

Anschließend wird ein Bestrahlungsfeld 45 festgelegt, mit dem das Zielvolumen 43 bestrahlt wird. Das eine Bestrahlungsfeld 45 ist dabei durch eine wählbare Einfallsrichtung gegeben, die festlegt, von welcher Richtung aus das Zielvolumen 43 bestrahlt wird. Bei einer Bestrahlung des Zielvolumens 43 mit Partikeln im Scanverfahren ergibt sich das Bestrahlungsfeld aus einer Vielzahl von nacheinander zu applizierenden Partikelstrahlen 47 (beamlets) aus derselben Einfallsrichtung, die sukzessive auf verschiedene Zielpunkte 49 im Zielvolumen 43 gerichtet werden. In Fig. 3 ist dies durch einige wenige im Zielvolumen 43 dargestellte Zielpunkte 49 symbolisiert.

Die Dichte-Heterogenität bei einem Bestrahlungsfeld 45 ist vornehmlich durch die Dichte-Heterogenität des Gebiets 51 gegeben, das in dem zu bestrahlenden Objekt 41 in Strahlrichtung vor dem Zielvolumen 43 liegt. Wenn beispielsweise ein anderes Bestrahlungsfeld gewählt wird, ändert sich die Dichte-Heterogenität, da vor dem Zielvolumen 43 ein anderes anatomisches Gebiet liegt, das eine andere Dichte-Heterogenität aufweist.

Nachdem das Bestrahlungsfeld 45 festgelegt worden ist, wird für dieses Bestrahlungsfeld 45 ein Maß ermittelt, das die Dichte-Heterogenität für dieses Bestrahlungsfeld 45 kennzeichnet. Das Maß kann beispielsweise für das gesamte Gebiet ermittelt werden, das durch das Bestrahlungsfeld 45 getroffen ist, oder auch lediglich für Teile des gesamten Gebietes. Beispielsweise ist es denkbar, das Maß lediglich für das in Strahlrichtung vor dem Zielvolumen liegende Gebiet 51 zu bestimmen, oder das Maß lediglich für Gebiete, die im Randbereich des Bestrahlungsfeldes liegen, etc. Im Gegensatz zu bekannten Verfahren, bei denen die laterale Dichte-Heterogenität für jeden einzelnen Partikelstrahl 47 (beamlets) ermittelt wird und in der Bestrahlungsplanung einfließt, wird hier ein Maß für ein Gebiet bestimmt, das durch eine Vielzahl von nacheinander zu applizierenden Partikelstrahlen getroffen wird.

Anschließend erfolgt die Wahl des Sicherheitssaumes 53 unter Zuhilfenahme des ermittelten Maßes. Optional kann zusätzlich der Einfallswinkel des Bestrahlungsfeldes 45 ebenfalls abhängig von dem Maß optimiert werden, beispielsweise derart, dass ein optimierter Einfallswinkel ein Bestrahlungsfeld 45 erzeugt, das eine geringe Dichte-Heterogenität aufweist. Der variabel wählbare und optimierbare Einfallswinkel ist durch den gebogenen Doppelpfeil 55 symbolisiert.

Die Wahl des Sicherheitssaumes 53 und/oder die Wahl des Einfallswinkels kann beispielsweise über einen funktionellen, in einer Rechnereinheit hinterlegten Zusammenhang geschehen, der auf Erfahrungswerten beruht, oder der auf Berechnungen gestützt ist. Derartige Berechnungen können beispielsweise die Fehleranfälligkeit eines Bestrahlungsplans gegenüber Unsicherheiten bei der Bestrahlung wie Bewegung des Zielvolumens, Lageunsicherheit, etc. einbeziehen. Alternativ ist es denkbar, über einen z.B. empirisch ermittelten, mathematischen Zusammenhang die Ausdehnung des Sicherheitssaumes abhängig von dem Maß zu ermitteln. Der Einfallswinkel kann beispielsweise mehrfach variiert werden, bis ein Einfallswinkel gefunden ist, zu dem das zugehörige Bestrahlungsfeld eine günstige Dichte-Heterogenität aufweist.

Die Wahl des Sicherheitssaumes 53 und/oder die Wahl des Einfallswinkels erfolgt üblicherweise zusätzlich unter Berücksichtigung von vorgegebenen Randbedingungen, beispielsweise unter Berücksichtigung der Aussparung von Organen, die nicht mit einer Dosis belastet werden sollen (so genannte "organs at risk"), etc.

Anhand von Fig. 4 ist die Planung einer Bestrahlung analog zu Fig. 3 erläutert, mit dem Unterschied, dass in Fig. 4 die Planung mit mehreren Bestrahlungsfeldern 45, 45' erfolgt. Auf eine Darstellung einzelner Partikelstrahlen mit Zielpunkten wurde der Übersichtlichkeit halber verzichtet.

Alternativ und/oder zusätzlich zu den Ausgestaltungen, die anhand von Fig. 3 erläutert worden sind, kann nun für jedes Bestrahlungsfeld 45, 45' ein Maß ermittelt werden, das jeweils die Dichte-Heterogenität in einem der Bestrahlungsfelder 45, 45' kennzeichnet. Diese Maße können nun dafür verwendet werden, eine Gewichtung der Bestrahlungsfelder 45, 45' untereinander zu bestimmen.

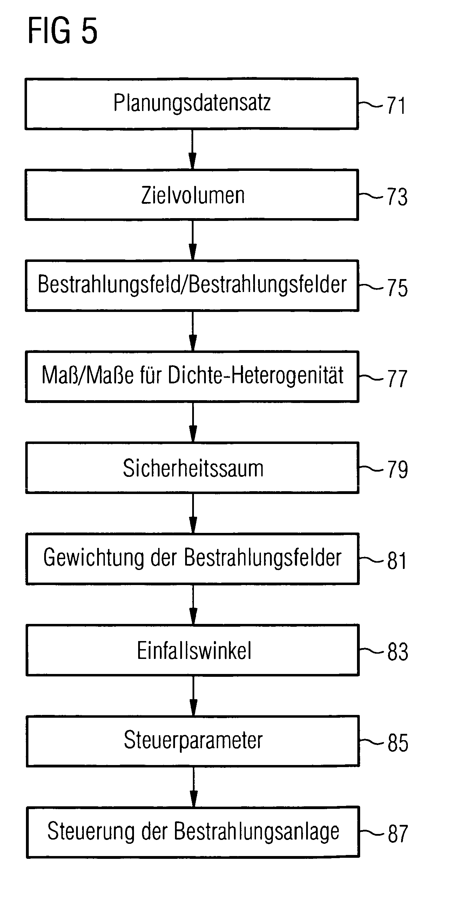

Fig. 5 zeigt eine schematische Übersicht über Verfahrensschritte, die bei einer Ausführungsform des Verfahrens ausgeführt werden können.

Zunächst wird ein Planungsdatensatz, in dem das zu bestrahlende Objekt abgebildet ist, bereitgestellt (Schritt 71). Anschließend wird das Zielvolumen in dem zu bestrahlenden Objekt bestimmt, das eine bestimmte Dosis erhalten soll (Schritt 73). Daraufhin werden ein oder mehrere Bestrahlungsfelder festgelegt (Schritt 75), und für dieses oder dieser Bestrahlungsfelder werden jeweils ein Maß ermittelt, das die Dichte-Heterogenität kennzeichnet, die für dieses oder diese Bestrahlungsfelder charakteristisch sind (Schritt 77). Dieses Maß wird dazu verwendet, um eine Gewichtung der Bestrahlungsfelder untereinander (Schritt 81) und/oder einen Sicherheitssaum für einzelne Bestrahlungsfelder zu bestimmen (Schritt 79). Zusätzlich kann der Einfallswinkel des oder der Bestrahlungsfelder in Abhängigkeit des Maßes gewählt werden (Schritt 83). Nachdem ein Bestrahlungsplan erstellt worden ist, können Steuerparameter zum Steuern der Anlage aus dem Bestrahlungsplan ermittelt werden (Schritt 85). Zur Durchführung der Bestrahlung gemäß dem Bestrahlungsplan wird die Bestrahlungsanlage mit den ermittelten Steuerparametern gesteuert (Schritt 87).

## Patentansprüche

1. Vorrichtung (25) zum Erstellen eines Bestrahlungsplans und zum Festlegen der Bestrahlung des Zielvolumens (43) durch eine Vielzahl von nacheinander zu applizierenden Partikelstrahlen (47), umfassend eine Rechnereinheit (27) mit einer Eingabevorrichtung (29) und einer Ausgabevorrichtung (29), welche Vorrichtung (25) ausgebildet ist zum Durchführen der folgenden Verfahrensschritte:
- Vorgeben eines Datensatzes, in welchem ein zu bestrahlendes Objekt (41) repräsentiert ist,
- Bestimmen eines zu bestrahlenden Zielvolumens (43) in dem Objekt (41),
- Ermitteln eines Maßes, das eine Dichte-Heterogenität für einen Bereich, der von einem geplanten Behandlungsstrahl getroffen wird, kennzeichnet, wobei der Bereich, dessen Dichte-Heterogenität durch das Maß gekennzeichnet wird, ein Gebiet umfasst, das von einer Mehrzahl der nacheinander zu applizierenden Partikelstrahlen (97) getroffen wird,
- Bestimmen eines Sicherheitssaumes (53) für das zu bestrahlende Zielvolumen (43) in Abhängigkeit des ermittelten Maßes.

2. Vorrichtung (25) nach Anspruch 1, wobei die Vorrichtung (25) weiterhin derart ausgebildet ist, dass der Bereich, dessen Dichte-Heterogenität durch das Maß kennzeichenbar ist, ein Gebiet (51) umfasst, das im Eintrittskanal des Behandlungsstrahls in Strahlrichtung vor dem Zielvolumen (43) liegt.

3. Vorrichtung (25) nach einem der Ansprüche 1 bis 2, wobei der Bereich, dessen Dichte-Heterogenität durch das Maß kennzeichenbar ist, das gesamte Gebiet umfasst, das durch den Behandlungsstrahl getroffen wird.

4. Vorrichtung (25) nach einem der Ansprüche 1 bis 3, wobei die Vorrichtung (25) zusätzlich ausgebildet ist zum Wählen eines Eintrittswinkel des Behandlungsstrahls in Abhängigkeit des Maßes.

5. Vorrichtung (25) nach einem der Ansprüche 1 bis 4, wobei die Vorrichtung (25) zusätzlich ausgebildet ist zum Überprüfen einer Dosisverteilung, die durch den geplanten Behandlungsstrahl gegeben ist, auf ihre Robustheit gegenüber einer räumlichen Ungenauigkeit bei der Dosis-Deposition.

6. Verfahren zur Erstellung eines Bestrahlungsplans für ein Scanverfahren mit einem Partikelstrahl, bei der die zu deponierende Dosis durch eine Vielzahl von nacheinander zu applizierenden, einzelnen Partikelstrahlen (47) erreicht wird, umfassend folgende Schritte:
- Vorgeben eines Datensatzes, in welchem ein zu bestrahlendes Objekt (41) repräsentiert ist,
- Bestimmen eines zu bestrahlenden Zielvolumens (43) in dem Objekt (41),
- Ermitteln eines Maßes, das eine Dichte-Heterogenität für einen Bereich, der von dem geplanten Behandlungsstrahl getroffen wird, kennzeichnet, wobei der Bereich, dessen Dichte-Heterogenität durch das Maß gekennzeichnet wird, ein Gebiet umfasst, das von einer Mehrzahl der nacheinander zu applizierenden Partikelstrahlen (47) getroffen wird,
- Bestimmen eines Sicherheitssaumes (53) für das zu bestrahlende Zielvolumen (43) in Abhängigkeit des ermittelten Maßes.

7. Verfahren nach Anspruch 6, wobei der Bereich, dessen Dichte-Heterogenität durch das Maß gekennzeichnet wird, ein Gebiet umfasst, das im Eintrittskanal des Behandlungsstrahls in Strahlrichtung vor dem Zielvolumen liegt.

8. Verfahren nach einem der Ansprüche 6 bis 7, wobei der Bereich, dessen Dichte-Heterogenität durch das Maß gekennzeichnet wird, das gesamte Gebiet umfasst, das durch den Behandlungsstrahl getroffen wird.

9. Verfahren nach einem der Ansprüche 6 bis 8, wobei zusätzlich ein Eintrittswinkel des Behandlungsstrahls in Abhängigkeit des Maßes gewählt wird.

10. Verfahren nach einem der Ansprüche 6 bis 9, wobei eine Dosisverteilung, die durch den geplanten Behandlungsstrahl gegeben ist, auf ihre Robustheit gegenüber einer räumlichen Ungenauigkeit bei der Dosis-Deposition überprüft wird.

## Claims

1. Device (25) for producing a radiation treatment plan and for establishing the irradiation of the target volume (43) by a multiplicity of particle beams (46) requiring to be applied successively, comprising a computer unit (27) having an input device (29) and an output device (29), which device (25) is embodied for implementing the following method steps:
- specifying a dataset in which an object (41) requiring to be irradiated is represented,
- determining a target volume (43) requiring to be irradiated within the object (41),
- ascertaining a metric identifying a density heterogeneity for a region that will be struck by a planned treatment beam, wherein the region whose density heterogeneity can be identified by the metric includes an area that will be struck by a plurality of the particle beams (47) requiring to be applied successively,
- determining as a function of the ascertained metric a safety margin (53) for the target volume (43) requiring to be irradiated.

2. Device (25) according to claim 1, wherein the device (25) is furthermore embodied such that the region whose density heterogeneity can be identified by the metric includes an area (51) situated in the treatment beam's entry channel in front of the target volume (43) in the beam direction.

3. Device (25) according to one of claims 1 to 2, wherein the region whose density heterogeneity can be identified by the metric includes the entire area that will be struck by the treatment beam.

4. Device (25) according to one of claims 1 to 3, wherein the device (25) is additionally embodied for selecting an entry angle of the treatment beam as a function of the metric.

5. Device (25) according to one of claims 1 to 4, wherein the device is additionally embodied for checking a dose distribution defined by the planned treatment beam for its robustness with respect to spatial imprecision in dose depositing.

6. Method for producing a radiation treatment plan for a scanning method with a particle beam in which the dose to be deposited by a multiplicity of individual particle beams (47) that are to be applied successively comprises the following steps::
- specifying a dataset in which an object (41) requiring to be irradiated is represented,
- determining a target volume (43) requiring to be irradiated within the object (41),
- ascertaining a metric identifying a density heterogeneity for a region that will be struck by the planned treatment beam, wherein the region whose density heterogeneity can be identified by the metric includes an area that will be struck by a plurality of the particle beams (47) requiring to be applied successively,
- determining as a function of the ascertained metric a safety margin (53) for the target volume (43) requiring to be irradiated

7. Method according to claim 6, wherein the region whose density heterogeneity is identified by the metric includes an area which is situated in the entry channel of the treatment beam in front of the target volume in the beam direction.

8. Device according to one of claims 6 to 7, wherein the region whose density heterogeneity is identified by the metric includes the entire area which will be struck by the treatment beam.

9. Method according to one of claims 6 to 8, wherein in addition an entry angle of the treatment beam is selected as a function of the metric.

10. Method according to one of claims 6 to 9, wherein a dose distribution defined by the planned treatment beam is checked for its robustness with respect to spatial imprecision in dose depositing.

## Revendications

1. Dispositif (25) d'établissement d'un plan d'irradiation et de fixation de l'irradiation du volume (43) cible par une pluralité de faisceaux (47) de particules à appliquer les uns après les autres, comprenant une unité (27) informatique ayant un dispositif (29) d'entrée et un dispositif (29) de sortie, lequel dispositif (25) est constitué pour effectuer les stades de procédé suivants :
- prescription d'un jeu de données dans lequel un objet (41) à irradier est représenté,
- détermination d'un volume (43) cible à irradier de l'objet,
- détermination d'une grandeur, qui caractérise une hétérogénéité de densité pour une région concernée par un faisceau de traitement planifié, la région, dont l'hétérogénéité de densité est **caractérisée par** la grandeur, comprenant une zone, qui est concernée par une pluralité des faisceaux (47) de particules à appliquer les uns après les autres,
- détermination d'un espace (53) de sécurité pour le volume (43) cible à irradier en fonction de la grandeur déterminée.

2. Dispositif (25) suivant la revendication 1, dans lequel le dispositif (25) est constitué en outre de manière à ce que la région, dont l'hétérogénéité de densité peut être **caractérisée par** la grandeur, comprenne une zone (51) qui se trouve dans le canal d'entrée du faisceau de traitement avant le volume (43) cible dans la direction du faisceau.

3. Dispositif (25) suivant l'une des revendications 1 à 2, dans lequel la région, dont l'hétérogénéité de densité peut être **caractérisée par** la grandeur comprend toute la zone qui est concernée par le faisceau de traitement.

4. Dispositif (25) suivant l'une des revendications 1 à 3, dans lequel le dispositif (25) est constitué en outre pour sélectionner un angle d'entrée du faisceau de traitement en fonction de la grandeur.

5. Dispositif (25) suivant l'une des revendications 1 à 4, dans lequel le dispositif (25) est constitué en outre pour contrôler la robustesse d'une répartition de dose, qui est donnée par le faisceau de traitement planifié, vis-à-vis d'une imprécision dans l'espace du dépôt de dose.

6. Procédé d'établissement d'un plan d'irradiation pour un procédé par balayage par un faisceau de particules, dans lequel on atteint la dose à déposer par une pluralité de faisceaux (47) de particules individuelles à appliquer les uns après les autres, comprenant les stades suivants :
- prescription d'un jeu de données dans lequel un objet (41) à irradier est représenté,
- détermination d'un volume (43) cible à irradier de l'objet,
- détermination d'une grandeur, qui caractérise une hétérogénéité de densité pour une région concernée par le faisceau de traitement planifié, la région, dont l'hétérogénéité de densité est **caractérisée par** la grandeur, comprenant une zone qui est concernée par une pluralité des faisceaux (47) de particules à appliquer les uns après les autres,
- détermination d'un espace (53) de sécurité pour le volume (43) cible à irradier en fonction de la grandeur déterminée.

7. Procédé suivant la revendication 6, dans lequel la région, dont l'hétérogénéité de densité est **caractérisée par** la grandeur, comprend une zone qui se trouve dans le canal d'entrée du faisceau de traitement avant le volume cible dans la direction du faisceau.

8. Procédé suivant l'une des revendications 6 à 7, dans lequel la région, dont l'hétérogénéité de densité est **caractérisée par** la grandeur, comprend toute la zone qui est concernée par le faisceau de traitement.

9. Procédé suivant l'une des revendications 6 à 8, dans lequel on choisit en plus un angle d'entrée du faisceau de traitement en fonction de la grandeur.

10. Procédé suivant l'une des revendications 6 à 9, dans lequel on contrôle la robustesse d'une répartition de dose, qui est donnée par le faisceau de traitement planifié, vis-à-vis d'une imprécision dans l'espace lors du dépôt de dose.
